# EUROPEAN PATENT SPECIFICATION

(11) **EP 2 111 855 B1**
(45) Date of publication and mention of the grant of the patent: **31.08.2016**
(21) Application number: 09166600.8
(22) Date of filing: 06.04.2007
(51) Int. Cl.: A61K 9/08, A61K 31/14, A61K 31/7024, A61P 11/00

(54) **Antibacterial compositions for the treatment of infections of the upper and lower airways**
Antibakterielle Zusammensatzung zur Behandlung von Infektion der oberen und unteren Luftwege
Compositions antibactériennes pour le traitement des infections des voise respiratoures supérieures et inférieures

(30) Priority: 13.04.2006 IT MI20060742
(43) Date of publication of application: 28.10.2009
(62) Divisional of application: 07007272.3
(73) Proprietor: CHIESI FARMACEUTICI S.p.A., 43100 Parma (IT)
(72) Inventor: Pattini, Patrizia, 20133 Milano (IT)
(74) Representative: Minoja, Fabrizio

(56) References cited:
- WO-A-95/26735
- US-A1- 2004 009 126
- US-A1- 2006 062 738
- PETRIGNI ET AL: PULM. PHARMACOL. THER, vol. 19, 2006, - 2006, pages 166-171,
- SMITH ET AL: J. CYSTIC FIBR, 2002, - 2002, pages s189-s193,
- NIKOLAIZIK ET AL: EUR.J.PEDRIATR, vol. 13, 2001, - 2001, pages 354-362,
- ELKINS ET AL: N.ENGL.J.MED, vol. 354, 2006, - 2006, pages 229-240,

## Description

This invention relates to compositions comprising hyaluronic acid and an hypertonic saline solution as suitable vehicle, which can be administered by inhalation.

### Prior art

*Pseudomonas aeruginosa* infection is one of the main unfavourable prognostic factors for patients suffering from cystic fibrosis (CF).

This disease is characterised by a gene defect that causes abnormal functioning of CFTR (cystic fibrosis transmembrane conductance regulator), a protein involved in the transmembrane transport of salts.

As a result of altered ion transport in the cell, cystic fibrosis is manifested by an alteration of the secretions of epithelial cells of bronchi, sinuses, pancreas, intestine, bile ducts and sweat glands.

Patients suffering from cystic fibrosis consequently produce mucus which is very thick and difficult to expectorate. The most serious problems generally affect the bronchi, where mucus viscosity makes it difficult for the bronchial cilia to remove inhaled particles. The mucus therefore fails to perform its main task of facilitating the removal of inhaled particles, thus generating conditions highly favourable to bacterial colonisation as a result of stagnation in the upper and lower airways. In particular, nearly all patients develop *Pseudomonas aeruginosa* infection, which becomes irreversible, and consequently chronic. As a result, CF patients are affected by a self-perpetuating trio of events that lead to an inexorable decline in the respiratory function.

This trio of events is known as obstruction - infection - inflammation. The mucus also protects bacteria against attack by antibiotics, which means that very high doses are needed to perform a bactericidal action.

In the case of aminoglycosides, the family of drugs which are most effective against this germ and Gram-negative bacteria in general, concentrations 25 times higher than the MIC are required to perform a bactericidal action, because concentrations 10 times greater than the MIC are considered only just bacteriostatic.

In order to administer repeated cycles of antibiotic treatment at the high concentrations required by chronic infection, antibiotic-based inhalation solutions for nebulisation in the lower airways only have been used for some time. These formulations enable the antibiotic to be conveyed to the site of the infection, allowing the use of high doses and at the same time reducing systemic exposure.

Bacterial colonisation by *Pseudomonas aeruginosa* and other pathogens takes place by direct contact, especially by inhaling particles of bacteria in suspension in the air. The upper airways play a crucial part in this colonisation process, representing a preferential access route for many germs.

The nose normally produces mucus, which has the function of trapping germs, pollen, dust and all the other particles in the air we breathe, preventing them from reaching the internal respiratory organs.

Extreme dryness of the mucus drastically reduces the efficacy of the nasal secretions, which inexorably become the ideal substrate for bacterial growth.

Before becoming chronic, *Pseudomonas* infection is manifested by a first infection, indicated by a first appearance of bacteria in the sputum; at this stage, a specifically-targeted antibiotic treatment can eradicate the germ and delay chronic infection, but the infection later returns intermittently, until it becomes chronic. At this stage, elimination of the germ from the nasal sinuses performs a significant preventive action against bacterial colonisation of the lower airways.

For this reason, specific antibacterial formulations directed towards these pathogens are highly desirable; such formulations should take account of the physiology of the nasal sinuses and of bronchi and perform an antibacterial and anti-inflammatory action thus preventing chronic infection and colonisation of the lower airways due to bacterial migration from the upper airways, reduce the bacteria count once the infection can no longer be eradicated, and at the same time reduce the correlated inflammation.

However, chronic bacterial colonisation, especially by Gram-negative germs, and *Pseudomonas* sp. in particular, is a possible and frequent event even in patients not suffering from CF, especially those with PCD (primary ciliary dyskinesia), severe chronic obstructive pulmonary disease (COPD), or patients with functional alterations of the nasal sinuses who are particularly predisposed to bacterial infection and the correlated inflammatory symptoms. At bronchial level, patients with alterations of the bronchial wall presenting sac-shaped areas (bronchiectasis) are particularly exposed to chronic infection.

Hyaluronic acid is a natural biopolymer belonging to the glycosaminoglycan (GAG) family, and is one of the main components of connective tissue matrix. The lungs, together with the skin and intestine, contain the largest amount of hyaluronic acid. Of the many known properties of hyaluronic acid, perhaps the main one is that it binds and retains a considerable amount of water.

Hyaluronic acid has also been used for its properties in numerous inflammation conditions. For example, its action on joint cartilage is known; hyaluronic acid is used by the intra-articular route to increase the viscous properties of the synovial fluid, and also because it performs a direct anti-inflammatory action, in both acute inflammation models and situations of chronic inflammation. The anti-inflammatory properties of hyaluronic acid are also used in dentistry, plastic surgery and dermatology.

The ability of hyaluronic acid to prevent bronchoconstriction in the stress test when administered by inhalation was also recently described (see e.g. G. Petrigni and L. Allegra, Pulm Pharmacol Ther. 2006, 19, 166-171).

The administration of antibiotics by inhalation is normally characterised by good systemic tolerability due to low systemic absorption, but is often accompanied by side effects such as bronchospasm due to local reactivity (see e.g. A.L. Smith, Journal of Cystic Fibrosis 1 2002 S189-S193; P.J. Cole, Journal of Chemotherapy 2001, 13, 354-362; W.H. Nikolaizik et al., Eur J Pediatr 1996, 155, 608-611).
This event is observed in some 10-15% of patients, who are consequently obliged to stop taking antibiotics, especially tobramycin.

In aerosol treatment of CF patients, treatment with a hypertonic solution (6-7% NaCl) has recently been suggested in addition to antibiotic treatment, with a view to hydrating the viscous secretions, thus improving their rheology and facilitating their elimination. This treatment has been found to improve the respiratory function (see e.g. M.R. Elkis et al., N Engl J Med 2006, 354, 229-240).

However, the administration of a hypertonic solution presents considerable problems, especially in terms of tolerability, as its inhalation causes inflammation of the respiratory mucosa, pharyngitis, laryngitis with reddening and local oedema, requiring discontinuance of the treatment, in a large number of patients (see e.g. M.R. Elkis et al., N Engl J Med 2006, 354, 229-240).

### DESCRIPTION OF THE INVENTION

It has now been found that the administration of a hypertonic solution, associated with hyaluronic acid or its salts, leads to better local tolerability, with a reduction in the inflammatory component affecting the mucosa of the airways, a lower risk of discontinuance of the treatment, and better compliance by patients affected by cystic fibrosis.

The hyaluronic acid usable according to the invention will preferably have a low or intermediate molecular weight.

The invention supplies formulations in sterile monodose ampoules of 5-7% hypertonic solution containing hyaluronic acid or a salt or ester thereof with a low molecular weight or an intermediate molecular weight at a concentration of between 0.01% and 1%, and preferably between 0.01% and 0.1%.

These formulations are rendered suitable for administration as aerosols in the lower airways by nebulisation through "Venturi effect" compressors such as Pari boy®, Pari Turbo boy®, Nebula®, etc. and suitable nebulisers, or through a vibration system such as Eflow rapid® or ultrasound nebulisation systems such as I-neb AAD.

The monodose formulations according to the invention will preferably be in the form of sterile aqueous solutions.

For the administration of aerosols in the lower airways, the solutions can be nebulised through a "Venturi effect" compressor such as Pari boy®, Pari Turbo boy®, Nebula®, etc. and suitable nebulisers, or a vibration system such as Eflow rapid®, ultrasound nebulisation systems, or systems such as I-neb AAD.

Hyaluronic acid, its salts (such as sodium salt) or esters have a molecular weight preferably between 50,000 and 200,000 Da, and are present in a concentration of between 0.01 and 5%. The mean aerodynamic diameter of the particles in the nebulised solutions is preferably between 2 and 5 microns.

The solutions to be nebulised, described above, can also be used for nebulisation in the upper airways, through a suitable system such as SinuNeb®.

The solution contains hyaluronic acid, its salts or esters, in the quantity of between 0.01% and 5% by weight, preferably between 0.05% and 1%, and more preferably between 0.05% and 0.5%.

The solution can be distributed between 5 ml monodose bottles containing the same composition as the multidose formulation.

The invention is illustrated in greater detail in the examples below.

### Example 1

### 4 ml Disposable ampoule

**Sterile solution**

| | |
|---|---|
| NaCl | 7% |
| Sodium hyaluronate | 0.05% |

### Example 2

### 4 ml Disposable ampoule

**Sterile solution**

| | |
|---|---|
| NaCl | 7% |
| Sodium hyaluronate | 0.02% |

## Claims

1. Inhalation formulations in sterile monodose ampoules of 5-7% hypertonic solution containing hyaluronic acid or a salt or ester thereof with a low molecular weight or an intermediate molecular weight at a concentration of between 0.01% and 1%.

2. Formulations according to claim 1 wherein the concentration of hyaluronic acid, salt or ester thereof ranges from 0.01% to 0.1 %.

3. Formulations as claimed in claim 1 or 2, administrable by the nasal route.

## Patentansprüche

1. Inhalationsformulierungen in sterilen Einzeldosisampullen von 5-7% hypertonischer Lösung, enthaltend Hyaluronsäure oder ein Salz oder einen Ester davon mit niedrigem Molekulargewicht oder mittlerem Molekulargewicht bei einer Konzentration von zwischen 0,01% und 1%.

2. Formulierungen gemäß Anspruch 1, wobei die Konzentration der Hyaluronsäure, des Salzes oder Esters davon von 0,01% bis 0,1% reicht.

3. Formulierungen gemäß Anspruch 1 oder 2, verabreichbar auf nasalem Weg.

## Revendications

1. Formulations pour inhalation en ampoules unidoses stériles de solution hypertonique à 5 à 7 % contenant de l'acide hyaluronique ou un sel ou un ester de celui-ci ayant un faible poids moléculaire ou un poids moléculaire intermédiaire à une concentration comprise entre 0,01 % et 1 %.

2. Formulations selon la revendication 1, dans lesquelles la concentration de l'acide hyaluronique, du sel ou de l'ester de celui-ci est comprise entre 0,01 % et 0,1 %.

3. Formulations selon la revendication 1 ou 2, administrable par la voie nasale.
